(19) Europäisches Patentamt
European Patent Office
Office européen des brevets

(11) Veröffentlichungsnummer: **0 045 955**
**B1**

# (12) EUROPÄISCHE PATENTSCHRIFT

(45) Veröffentlichungstag der Patentschrift: **31.08.83**

(51) Int. Cl.³: **C 07 D 271/10**, A 61 K 31/41

(21) Anmeldenummer: **81106178.7**

(22) Anmeldetag: **07.08.81**

## (54) Alkyl- und cycloalkylsubstituierte 2-(2-(2-Hydroxy-3-tert.-butylamino-propoxy)-5-acylamino-phenyl)-1,3,4-oxadiazole, Verfahren zu ihrer Herstellung und diese Verbindungen enthaltende pharmazeutische Zubereitungen.

(30) Priorität: **13.08.80 DE 3030530**

(43) Veröffentlichungstag der Anmeldung: **17.02.82 Patentblatt 82/7**

(45) Bekanntmachung des Hinweises auf die Patenterteilung: **31.08.83 Patentblatt 83/35**

(84) Benannte Vertragsstaaten: **AT BE CH DE FR GB IT LI NL SE**

(56) Entgegenhaltungen:
**EP-A-0 034 276**
**DE-A-2 811 638**

(73) Patentinhaber: **Beiersdorf Aktiengesellschaft, Unnastrasse 48, D-2000 Hamburg 20 (DE)**

(72) Erfinder: **Cohnen, Erich, Dr., Dipl.-Chem., Heilwigstrasse 25, D-2000 Hamburg 20 (DE)**

**EP 0 045 955 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Alkyl- und cycloalkylsubstituierte 2-[2-(2-Hydroxy-3-tert.-butylamino-propoxy)-5-acylamino-phenyl]-1,3,4-oxadiazole, Verfahren zu ihrer Herstellung und diese Verbindungen enthaltende pharmazeutische Zubereitungen

Gegenstand der Erfindung sind neue 2-[2-(2-Hydroxy-3-tert.-butylamino-propoxy)-5-acylamino-phenyl]-1,3,4-oxadiazole der allgemeinen Formel I

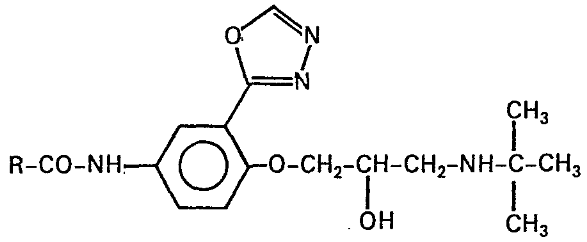

(I)

in der R eine geradkettige oder verzweigtkettige Alkylgruppe mit 1 bis 17 Kohlenstoffatomen oder eine Cycloalkylgruppe mit 3 bis 6 Kohlenstoffatomen darstellt, sowie deren physiologisch verträgliche Säureadditionssalze, Verfahren zu ihrer Herstellung sowie ihre Verwendung in pharmazeutischen Präparaten.

Die hier gewählte Definition für R bedeutet sowohl geradkettige als auch verzweigtkettige aliphatische, 1 bis 17 Kohlenstoffatome enthaltende Kohlenwasserstoffreste, wie z.B. die Methylgruppe, Äthylgruppe, n-Propylgruppe, Isopropylgruppe, n-Butylgruppe, sek.-Butylgruppe, Isobutylgruppe, tert.-Butylgruppe, n-Pentylgruppe, Isopentylgruppe, Neopentylgruppe, Amylgruppe, n-Hexylgruppe, Isohexylgruppen und die verzweigten Hexylgruppen mit quartärem Kohlenstoffatom sowie die n-, iso- oder quartären Heptyl-, Octyl-, Nonyl-, Decylgruppen und die entsprechenden Homologen höherer Kohlenstoffzahl mit bis zu 17 Kohlenstoffatomen.

Besonders bevorzugt wird die n-Hexylgruppe.

Geeignete Cycloalkylsubstituenten sind die Cyclopropyl-, Cyclobutyl-, Cyclopentyl- und Cyclohexylgruppe.

Die erfindungsgemässen Verbindungen und ihre Säureadditonssalze besitzen eine überlegene therapeutische Wirksamkeit. Sie zeichnen sich in überraschender Weise durch eine spezifische $\beta_1$-adrenolytische und blutdrucksenkende Wirkung aus und können daher sehr vorteilhaft zur Behandlung von Angina pectoris, Hypertonie und Arrhythmien eingesetzt werden.

Die Verbindungen können oral oder parenteral angewendet werden. Eine Dosierung von 0,4–5 mg/kg i.v. senkt beim Kaninchen die isoprenalinbedingte Tachycardie um 50%. Die Dosierung beim Menschen beträgt 50–200 mg pro Tag, wobei die Verabreichung vorzugsweise ein- bis zweimal täglich erfolgt.

Zur Hypertoniebehandlung werden bevorzugt Dosen von 50–100 mg ein- bis zweimal täglich verabreicht. Die Behandlung der Angina pectoris erfolgt vorzugsweise mit 50–100 mg zweimal pro Tag. Zur Bekämpfung von Herzrhythmusstörungen werden 100 mg ein- bis zweimal täglich verabreicht.

Die meisten im Handel befindlichen sowie in der Struktur ähnliche $\beta$-Adrenolytika beeinflussen sowohl $\beta_1$- wie $\beta_2$-Rezeptoren, d.h. sie hemmen den Einfluss des Sympathicus auf das Herz ebenso wie auf die peripheren Gefässe oder die Bronchialmuskulatur. Die Blockierung der $\beta_2$-Rezeptoren führt zu einer Tonuserhöhung der glatten Muskulatur mit Bronchospasmen und Widerstandserhöhung der peripheren Gefässe. Die erfindungsgemässen Substanzen blockieren dagegen überraschenderweise vorwiegend die $\beta_1$-Rezeptoren des Herzens. Diese Cardioselektivität ist bei akuten und chronisch obstruktiven Bronchialerkrankungen von besonders grosser Bedeutung, da $\beta_2$-rezeptorenblockierende Substanzen aufgrund ihrer bronchokonstriktorischen Wirkung bei Bronchialerkrankungen (Asthma) nicht verabreicht werden dürfen, eine Behandlung der obengenannten Krankheiten mit $\beta$-Adrenolytika aber erforderlich ist.

Eine weitere unspezifische Eigenschaft vieler bekannter $\beta$-Adrenolytica ist ihre lokalanästhetische Wirkung, die zu cardiodepressiven Effekten (Kontraktilitätshemmung) führt. Die agonistische Wirkkomponente, die auch als intrinsische Aktivität (ISA) bezeichnet wird, ist ebenfalls eine unerwünschte Nebenwirkung einer Reihe von $\beta$-Blokkern, die sich bei der Hypertoniebehandlung nachteilig bemerkbar macht. Die erfindungsgemässen Verbindungen zeichnen sich dagegen durch das Fehlen sowohl der lokalanästhetischen als auch der agonistischen Wirkung aus, wodurch das Ausmass der genannten Nebenwirkungen sehr erheblich vermindert wird.

Aufgabe der Erfindung ist es, neue, spezifisch am Herz wirksame Verbindungen aufzuzeigen, die diese genannten nachteiligen Nebenwirkungen nicht oder nur in sehr geringem Mass aufweisen. Zur Lösung dieser Aufgabe wurden umfangreiche Forschungsarbeiten seitens des Erfinders durchgeführt, in der Absicht, unter Berücksichtigung der Vielzahl ähnlich strukturierter Verbindungen, die erfindungsgemässen, überraschend selektiv- und hochwirksamen Verbindungen aufzufinden.

Die erfindungsgemässen Verbindungen besitzen diese hervorragenden Eigenschaften gegenüber anderen, in der Struktur ähnlichen Verbindungen, wie in der deutschen Offenlegungsschrift DE-A-28 11 638 angegeben, in nicht vorhersehbarer Weise. Man nimmt an, dass diese besonderen, spezifischen Wirkungen von der

speziellen Struktur der erfindungsgemässen Verbindungen abhängen.

Diese überraschende überlegene Wirksamkeit der neuen Verbindungen ist unter anderem auf die ortho-Stellung der 3-tert.-Butylamino-2-hydroxy-1-propoxy-Seitenkette des Phenylrings zusammen mit der meta-Stellung der erfindungsgemässen Acylaminogruppen zum 1,3,4-Oxadiazol-Ring zurückzuführen.

Ein weiterer erheblicher Vorteil der erfindungsgemässen Verbindungen ist ihre sehr grosse Halbwertszeit.

Besonders wirksam und vorteilhaft sind erfindungsgemässe 2-[2-(2-Hydroxy-3-tert.-butylamino-propoxy)-5-acylamino-phenyl]-1,3,4-oxadiazole der allgemeinen Formel I, in der ein aliphatischer Alkylsubstituent R in der 5-Acylgruppe 1, 5, 6, 7, 8, 9 oder 10 Kohlenstoffatome enthält und deren Salze.

Von diesen Verbindungen wird die folgende Auswahl der Verbindungen bevorzugt, in denen R einen geradkettigen aliphatischen Alkylrest mit 1, 5, 6, 7, 8, 9 oder 10 Kohlenstoffatomen bedeutet. Es sind die Verbindungen:

a) 2-[2-(2-Hydroxy-3-tert.-butylamino-propoxy)-5-acetamino-phenyl]-1,3,4-oxadiazol
b) 2-[2-(2-Hydroxy-3-tert.-butylamino-propoxy)-5-n-hexanoylamino-phenyl]-1,3,4-oxadiazol
c) 2-[2-(2-Hydroxy-3-tert.-butylamino-propoxy)-5-n-heptanoylamino-phenyl]-1,3,4-oxadiazol
d) 2-[2-(2-Hydroxy-3-tert.-butylamino-propoxy)-5-n-octanoylamino-phenyl]-1,3,4-oxadiazol
e) 2-[2-(2-Hydroxy-3-tert.-butylamino-propoxy)-5-n-nonanoylamino-phenyl]-1,3,4-oxadiazol
f) 2-[2-(2-Hydroxy-3-tert.-butylamino-propoxy)-5-n-decanoylamino-phenyl]-1,3,4-oxadiazol
g) 2-[2-(2-Hydroxy-3-tert.-butylamino-propoxy)-5-n-undecanoylamino-phenyl]-1,3,4-oxadiazol

Die bevorzugten selektiv wirksamen Verbindungen innerhalb dieser Auswahl sind die Verbindungen a), c), d), e) und f), wobei die Verbindungen c) und d) sich durch ihre überlegene Selektivität auszeichnen.

Von diesen beiden Verbindungen wird die Verbindung c), 2-[2-(2-Hydroxy-3-tert.-butylamino-propoxy)-5-n-heptanoyl-aminophenyl]-1,3,4-oxadiazol und deren Salze, besonders bevorzugt, da sie sich ausserdem durch eine grosse Halbwertszeit auszeichnet.

In der folgenden Tabelle 1 sind die wichtigen pharmakologischen Eigenschaften einiger der überlegen wirksamen erfindungsgemässen Verbindungen mit der angegebenen Bedeutung von R gemäss der allgemeinen Formel I sowie der bekannten strukturähnlichen, als Vergleichssubstanzen dienenden Verbindungen Acebutolol und 2-[2-(2-Hydroxy-3-tert.-butylamino-propoxy)-phenyl]-1,3,4-oxadiazol aufgeführt. Die letztgenannte Verbindung ist aus der deutschen Offenlegungsschrift DE-A 28 11 638 bekannt.

Tabelle 1

| Verbindung des Beisp. Nr. | R | $pA_2$ (Atrium) | $pA_2$ (Trachea) | Cardio-selektivität antilog $pA_2(A)-pA_2(T)$ | ISA % Iso prenalin-Wirkung | lokal-anäst. Wirkung | $t_{1/2}$ (h) |
|---|---|---|---|---|---|---|---|
| 1 | $CH_3$ | 6,58 | 5,38 | 17 | 0 | 0 | 5 |
| 2 | $CH_3(CH_2)_5-$ | 7,3 | 5,9 | 25 | 0 | 0 | 3,5 |
| 4 | $CH_3CH_2)_6-$ | 7,4 | 5,9 | 32 | 0 | 0 | - |
| 6 | $CH_3(CH_2)_8-$ | 7,1 | 5,8 | 20 | 0 | 0 | - |
| Acebutolol* (Vergleichssubstanz 1) | | 6,29 | 5,01 | 19 | 27 | vorhanden | 1,6 |
| 2-[2-(2-Hydroxy-3-tert.-butylamino-propoxy)-phenyl]-1,3,4-oxadiazol (Vergleichssubstanz 2) | | 7,3 | 6,8 | 3 | 79 | 0 | - |

* 1-(2-Acetyl-4-n-butyramidophenoxy)-2-hydroxy-3-isopropylamino-propan-hydrochlorid

Die in der Tabelle 1 genannten Begriffe haben die folgenden, auch aus der Literatur (B.J. Clark: «Pharmacology of β-Adrenoceptor blocking agents» in «β-Adrenoceptor blocking agents», ed. P.R. Saxena und R.P. Forsyth) bekannten Bedeutungen:

$pA_2$: Ausdruck für die Stärke eines β-Adrenoceptorenblockers. Entspricht dem negativen Logarithmus der molaren Konzentration eines Antagonisten, die eine Verdoppelung der molaren Konzentration des Agonisten erfordert, um einen vorgegebenen Effekt am isolierten Gewebe zu erzielen. Als Testgewebe dienen der Herz-Vorhof und die Trachea des Meerschweinchens.

Cardioselektivität: antilog $pA_2$(Atrium)-$pA_2$ (Trachea)

ISA: «Intrisic sympathomimetic activity» ($\beta_1$-agonistische Wirkung). Angegeben ist der prozentuale Anstieg der Herzfrequenz, die durch eine Substanz induziert wird, bezogen auf die maximale Reaktion von Isoprenalin (100%). Die Messungen werden an mit Reserpin vorbehandelten Ratten durchgeführt, die keinen sympathischen Tonus mehr aufweisen.

$t_{1/2}$: Zeit, nach der die β-adrenolytische Wirkung

einer Substanz am Kaninchen nach i.v. Applikation auf die Hälfte abgenommen hat.

Aus der Tabelle 1 ist die überlegene pharmakologische Wirksamkeit der erfindungsgemässen Verbindungen ersichtlich. Sie besitzen im Gegensatz zur Vergleichssubstanz 1 keine nachteilige Isoprenalinwirkung, während die Cardioselektivität vergleichbar gut oder besser ist. Gegenüber der Vergleichssubstanz 2 zeichnen sich die erfindungsgemässen Verbindungen durch ihre überragende Cardioselektivität aus. Die hohe Isoprenalinwirkung der Vergleichssubstanz ist dagegen unvorteilhaft.

Die Verbindung 2-[2-(2-Hydroxy-3-tert.-butylamino-propoxy)-5-n-heptanoyl-aminophenyl]-1,3,4-oxadiazol und deren Salze zeichnen sich durch hohe Selektivität und eine gute Halbwertszeit aus.

Gemäss der Erfindung werden pharmazeutische Zusammensetzungen geschaffen, die eine Verbindung der Formel I oder deren pharmazeutisch verträgliche Salze, zusammen mit einem pharmazeutisch verträglichen Verdünnungsmittel oder Träger enthalten.

Die Verbindungen gemäss der Erfindung können mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können oral in Form von Tabletten, Dragees, Sirups, Suspensionen und Flüssigkeiten, oder parenteral in Form von Lösungen oder Suspensionen verabreicht werden. Oral zu verabreichende Präparate können ein oder mehrere Zusätze, wie Süssungsmittel, Aromatisierungsmittel, Farbstoffe und Konservierungsmittel, enthalten. Tabletten können den Wirkstoff mit üblichen, pharmazeutisch verträglichen Hilfsmitteln vermischt enthalten, z.B. inerten Verdünnungsmitteln wie Calciumcarbonat, Natriumcarbonat, Lactose und Talk, Granulierungsmitteln und Mitteln, die den Zerfall der Tabletten bei oraler Verabreichung fördern, wie Stärke oder Alginsäure, Bindemitteln wie Stärke oder Gelatine, Gleitmitteln wie Magnesiumstearat, Stearinsäure und Talk.

Geeignete Trägerstoffe sind beispielsweise Milchzucker (Lactose), Gelatine, Maisstärke, Stearinsäure, Äthanol, Propylenglycol Äther des Tetrahydrofurfurylalkohols und Wasser.

Die Tabletten können nach bekannten Arbeitsweisen überzogen werden, um den Zerfall und die Resorption im Magen-Darmtrakt zu verzögern, wodurch die Aktivität des Wirkstoffs sich über eine längere Zeitspanne erstrecken kann. Ebenso kann in den Suspensionen der Wirkstoff mit Hilfsmitteln vermischt sein, die für die Herstellung solcher Zusammensetzungen üblich sind, z.B. Suspendiermitteln wie Methylcellulose, Tragacanth oder Natriumalginat, Netzmitteln wie Lecithin, Polyoxyäthylenstearat und Polyoxyäthylensorbitanmonooleat und Konservierungsmitteln wie Äthylparahydroxybenzoat. Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert. Die pharmazeutischen Präparate können den Wirkstoff in einer Menge von 0,1 bis 90%, insbesondere 1 bis 90%, enthalten, wobei der Rest ein Trägerstoff oder Zusatzstoff ist. Im Hinblick auf die Herstellung und Verabreichung werden feste Präparate, wie Tabletten und Kapseln, bevorzugt. Vorzugsweise enthalten die Präparate den Wirkstoff in einer Menge von 50 bis 200 mg entsprechend der bevorzugten Tagesdosis.

Das Verfahren zur Herstellung der Verbindungen der Formel I ist dadurch gekennzeichent, dass man substituierte Oxadiazole der allgemeinen Formel II

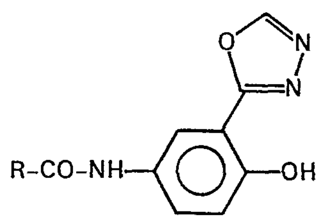

(II)

in der R die oben angegebene Bedeutung hat, mit Epichlorhydrin oder Epibromhydrin zu einem Gemisch von Verbindungen der allgemeinen Formeln III und IV umsetzt

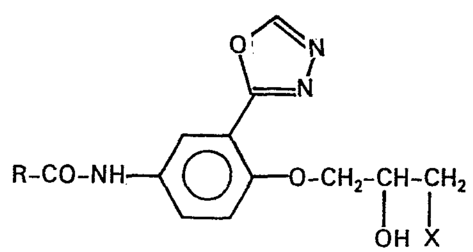

(III)

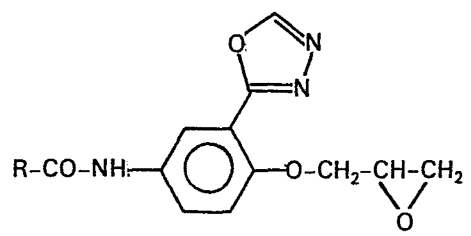

(IV)

worin R die angegebene Bedeutung hat und X ein Chlor- oder Bromatom darstellt, und dieses anschliessend mit tert.-Butylamin zu Verbindungen der Formel I umsetzt.

Die erfindungsgemässen freien Basen werden gegebenenfalls mit Säuren nach üblichen Verfahren in die entsprechenden Säureadditionssalze überführt. Geeignete Säuren sind beispielsweise Chlor- oder Bromwasserstoffsäure, Schwefel-, Oxal-, Fumar- oder Maleinsäure, die z.B. in alkoholischer oder ätherischer Lösung mit der Base umgesetzt werden.

Die verfahrensgemäss eingesetzten Verbindungen der Formel II können aus den entsprechenden Hydroxybenzoesäureestern hergestellt werden:

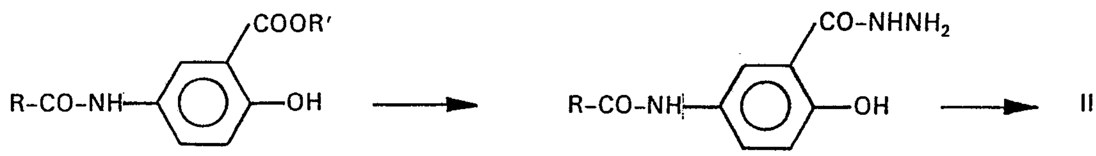

(V) (VI)

R hat hierin die oben angegebene Bedeutung und R' bedeutet eine niedere Alkylgruppe.

Eine bevorzugte Ausführungsform dieses Verfahrens umfasst folgende Stufen:

a) Reaktion des entspechenden Esters V mit Hydrazinhydrat in Methanol oder Äthanol in der Siedehitze zu den Hydraziden VI
b) Cyclisierung der Verbindungen VI mit einem Orthoameisensäureester zu den 1,3,4-Oxadiazolen II.

Die Ausgangsverbindungen V sind bekannt oder nach bekannten, in der Literatur beschriebenen Methoden zugänglich. Sie können durch Acylierung der Aminosalicylsäureester oder Umacylierung der bekannten Acylaminosalicylsäureester erhalten werden.

Die Reaktion der Verbindung II mit Epichlor- oder Epibromhydrin zu den Substanzen III und IV wird vorzugsweise bei Temperaturen zwischen 20 bis 50°C in Epichlor- bzw. Epibromhydrin ausgeführt. Das Gemisch der Verbindungen III und IV oder die Einzelkomponenten werden nach Reinigung mit tert.-Butylamin bei Raumtemperatur zu den erfindungsgemässen Verbindungen I umgesetzt, wobei ein Alkohol als Lösungsmittel dient.

Die folgenden Beispiele dienen zur Erläuterung der Erfindung:

Beispiel 1

2-[2-(2-Hydroxy-3-tert.-butylamino-propoxy)-5-acetamido-phenyl]-1,3,4-oxadiazol

a) 41,8 g (0,2 M) 5-Acetamido-salicylsäuremethylester werden in 150 ml Äthanol suspendiert und mit 12 ml (0,24 M) Hydrazinhydrat 4 Stunden zum Sieden erhitzt. Nach Abkühlen werden 24 g reines Hydrazid durch Filtration isoliert.

b) 24 g 5-Acetamido-salicylsäurehydrazid werden mit 200 ml Orthoameisensäuretriäthylester in 200 ml Dimethylformamid 24 Stunden unter Rückfluss erhitzt. Nach Abdestillieren des Orthoesters und Dimethylformamids wird der Rückstand mit Aceton versetzt und das kristalline 2-(2-Hydroxy-5-acetamido-phenyl)-1,3,4-oxadiazol abgesaugt.
Fp. 235–236°C, Ausbeute: 14 g.

c) 3 g 2-(2-Hydroxy-5-acetamido-phenyl)-1,3,4-oxadiazol werden in 30 ml Epibromhydrin unter Zusatz von 2 Tropfen Piperidin 12 Stunden auf 50°C erwärmt. Nach Abtrennung des nicht umgesetzten Ausgangsprodukts wird das Bromhydrin abdestilliert und der Rückstand mit Diisopropyläther verrieben. Man erhält 0,5 g 2-[2-(2,3-Epoxypropoxy)-5-acetamidophenyl]-1,3,4-oxadiazol. Fp. 173–175°C.

d) 0,4 g der Verbindung 1c) werden in 30 ml Methanol und 30 ml tert.-Butylamin 15 Stunden bei 20–25°C gerührt. Die Lösung wird eingedampft und der Rückstand aus Toluol umkristallisiert: 2-[2-(2-Hydroxy-3-tert.-butylamino-propoxy)-5-acetamidophenyl]-1,3,4-oxadiazol.
Fp. 157–160°C, Ausbeute: 0,5 g.

0,4 g der Base werden in Äthanol gelöst und 65 mg Fumarsäure zugesetzt. Nach Zusatz von Diisopropyläther erhält man 300 mg des Fumarats des 2-[2-(2-Hydroxy-3-tert.-butylamino-propoxy)-5-acetamido-phenyl]-1,3,4-oxadiazols.
Fp. 217–219°C (Z.).

Beispiel 2

2-[2-(2-Hydroxy-3-tert.-butylamino-propoxy)-5-n-heptanoylamino-phenyl]-1,3,4-oxadiazol

a) 35 g (0,12 M) 5-n-Heptanoylamino-salicylsäuremethylester werden in 200 ml Äthanol suspendiert und mit 11,6 ml (0,24 M) Hydrazinhydrat 6 Stunden zum Sieden erhitzt. Nach Abkühlen werden 28,2 g des Hydrazids durch Filtration isoliert.
Fp. 204–206°C.

b) 28 g (0,1 M) 5-n-Heptanoylamino-salicylsäurehydrazid werden mit 250 ml Orthoameisensäuretriäthylester 3 Stunden unter gleichzeitiger Abdestillation von Äthanol erhitzt. Nach Abkühlen kristallisieren 14,1 g des Oxadiazols aus.
Fp. 207–209°C.

c) 1,45 g 2-(2-Hydroxy-5-n-heptanoylamino-phenyl)-1,3,4-oxadiazol werden in 50 ml Butanon gelöst und nach Zusatz von 0,7 g Kaliumcarbonat und 0,85 ml Epibromhydrin 8 Stunden zum Sieden erhitzt. Nach Abtrennung des nicht umgesetzten Ausgangsprodukts wird das Lösungsmittel abdestilliert und der Rückstand durch HPLC an Kieselgel gereinigt. Man erhält 0,8 g 2-[2-(2,3-Epoxipropoxy)-5-n-heptanolyaminophenyl]-1,3,4-oxadiazol.
Fp. 135–136°C.

d) 0,7 g 2-[(2,3-Epoxipropoxy)-5-n-heptanoylaminophenyl]-1,3,4-oxadiazol, werden in 20 ml t-Butanol und 20 t-Butylamin 20 Stunden bei Raumtemperatur gerührt. Nach Abdampfen des Lösungsmittels wird der Rückstand in wenig Äthanol gelöst und durch Zugabe von Oxalsäure (0,12 g) das Oxalat des 2-[2-(2-Hydroxy-3-tert.-butylamino-propoxy)-5-n-heptanoylamino-phenyl]-1,3,4-oxadiazols gefällt (Oxalat + 0,5 $H_2O$).
Fp. 152–153°C (Z.).

Analog Beispiel 1 und 2 wurden folgende erfindungsgemässe Verbindungen erhalten:

Beispiel 3

2-[2-(2-Hydroxy-3-tert.-butylamino-propoxy)-5-n-hexanoylamino-phenyl]-1,3,4-oxadiazol
Fp. 140–142°C.

Beispiel 4

2-[2-(2-Hydroxy-3-tert.-butylamino-propoxy)-5-n-octanoylamino-phenyl]-1,3,4-oxadiazol
Fp. 182–183°C (Oxalat + 0,5 $H_2O$).

Beispiel 5

2-[2-(2-Hydroxy-3-tert.-butylamino-propoxy)-5-n-nonanoylamino-phenyl]-1,3,4-oxadiazol
Fp. 130–132°C.

Beispiel 6

2-[2-(2-Hydroxy-3-tert.-butylamino-propoxy)-5-n-decanoylamino-phenyl]-1,3,4-oxadiazol
Fp. 135–136°C.

Beispiel 7

2-[2-(2-Hydroxy-3-tert.-butylamino-propoxy)-5-n-undecanoylamino-phenyl]-1,3,4-oxadiazol
Fp. 130–132°C.

Als Zwischenprodukte obengenannter Verbindungen wurden, ausgehend von den entsprechenden Acylamino-salicylsäureestern (die durch Acylierung der Aminosalicylsäureester oder Umacylierung bekannter Acylaminosalicylsäureester hergestellt werden können), analog Beispiel 1 und 2 die folgenden Verbindungen erhalten:

5-n-Hexanoylamino-salicylsäurehydrazid
Fp. 206–208°C
5-n-Octanoylamino-salicylsäurehydrazid
Fp. 202–204°C
5-n-Nonanoylamino-salicylsäurehydrazid
Fp. 204–206°C
5-n-Decanoylamino-salicylsäurehydrazid
Fp. 203–205°C
5-n-Undecanoylamino-salicylsäurehydrazid
Fp. 205–206°C
2-(2-Hydroxy-5-n-hexanolyamino-phenyl]-1,3,4-oxadiazol
Fp. 210–211°C
2-(2-Hydroxy-5-n-octanoylamino-phenyl]-1,3,4-oxadiazol
Fp. 206–207°C
2-(2-Hydroxy-5-n-nonanoylamino-phenyl]-1,3,4-oxadiazol
Fp. 204–205°C
2-(2-Hydroxy-5-n-decanoylamino-phenyl]-1,3,4-oxadiazol
Fp. 204–207°C
2-(2-Hydroxy-5-n-undecanoylamino-phenyl)-1,3,4-oxadiazol
Fp. 206–208°C

Beispiel 8

2-[2-(2-Hydroxy-3-tert.-butylamino-propoxy)-5-isobutanoylaminophenyl]-1,3,4-oxadiazol (R = isopropyl)

a) 24 g (0,096 M) 5-Isobutanoylamino-salicylsäureäthylester werden in 100 ml Äthanol suspendiert und mit 7,3 ml (0,15 M) Hydrazinhydrat 2 Stunden zum Sieden erhitzt. Nach Abkühlen werden 21,5 g des Hydrazids durch Filtration isoliert.
Fp. 228°C (Z.).

b) 20,0 g (0,08 M) 5-Isobutanoylamino-salicylsäurehydrazid werden mit 100 ml Orthoameisensäuretriäthylester unter Zusatz von 25 ml Dimethylformamid 5 Stunden unter Rückfluss erhitzt, wobei das entstehende Äthanol abdestilliert wird. Nach Abkühlen kristallisieren 12,3 g des Oxadiazols aus.
Fp. 244–246°C.

c) 9,0 g 2-(2-Hydroxy-5-isobutanoylamino-phenyl]-1,3,4-oxadiazol werden mit 90 ml Epichlorhydrin unter Zusatz von katalytischen Mengen Piperidin 2 Stunden zum Sieden erhitzt. Nach Abdestillation des überschüssigen Chlorhydrins und Reinigung des Epoxids durch HPLC an Kieselgel, erhält man durch Umsetzung mit tert.-Butylamin in tert.-Butanol bei Raumtemperatur und nach üblicher Aufarbeitung das Oxalat des 2-[2-(2-Hydroxy-3-tert.-butylamino-propoxy)-5-isobutanoylamino-phenyl]-1,3,4-oxadiazols.
Fp. 178–183°C.

Analog zu vorstehenden Beispielen wurden die folgenden erfindungsgemässen Verbindungen erhalten:

Beispiel 9

2-[2-(2-Hydroxy-3-tert.-Butylamino-propoxy)-5-cyclopropanoylamino-phenyl]-1,3,4-oxadiazol (R = cyclopropyl)
Fp. 115–117°C.

Als Zwischenprodukte zu der obengenannten Verbindung wurden analog Beispiel 1, 2 und 8 erhalten:

5-Cyclopropanoylamino-salicylsäureäthylester
Fp. 154–158°C.
5-Cyclopropanoylamino-salicylsäurehydrazid
Fp. 234–236°C.
2-(2-Hydroxy-5-cyclopropanoylamino-phenyl)-1,3,4-oxadiazol
Fp. 272–274°C (Z.).

Beispiel 10

2-[2-(2-Hydroxy-3-tert.-butylamino-propoxy)-5-cyclohexanoylamino-phenyl]-1,3,4-oxadiazol (R = cyclohexyl),
Fp. 110–114°C.

Als Zwischenprodukte obengenannter Verbindung wurden erhalten:

5-Cyclohexanoylamino-salicylsäureäthylester
Fp. 184–186°C.
5-Cyclohexanoylamino-salicylsäurehydrazid
Fp. 233–235°C (Z.).
2-(2-Hydroxy-5-cyclohexanoylamino-phenyl)-1,3,4-oxadiazol
Fp. 239–241°C.

Analog vorstehenden Beispielen wurden die folgenden erfindungsgemässen Verbindungen synthetisiert:

| Beispiel | R | Fp. °C | Salz |
|---|---|---|---|
| 11 | $CH_3CH_2$- | 178–181 | Oxalat + 1 $H_2O$ |
| 12 | $CH_3(CH_2)_3$- | 178–181 | Oxalat + 0,5 $H_2O$ |
| 13 | $CH_3(CH_2)_{10}$ | 126–128 | Base |
| 14 | $CH_3(CH_2)_{16}$ | 124–125 | Base |

Die Herstellung von Arzneimitteln unter Verwendung einer Verbindung gemäss der Erfindung wird nachstehend anhand eines Beispiels näher erläutert.

Beispiel 15

Herstellung von Tabletten

Tabletten, welche die nachstehend angegebenen Bestandteile enthalten, werden nach bekannten Arbeitsweisen hergestellt. Diese sind für die Behandlung von Hypertonie in einer Dosierungsmenge von 50 mg ein- bis zweimal täglich, für die Behandlung von Angina pectoris mit einer Menge von 75 mg zweimal täglich und zur Behandlung von Arrhythmien (Herzrhythmusstörungen) mit einer Menge von ein- bis zweimal 100 mg täglich geeignet.

| Bestandteile | Gewicht (mg) Tablette A | Gewicht (mg) Tablette B |
|---|---|---|
| 2-[2-(2-Hydroxy-3-tert.-butylamino-propoxy)-5-n-heptanoylamino-phenyl]-1,3,4-oxadiazol | 50 | 75 |
| Tragacanth | 10 | - |
| Lactose | 297,5 | 300 |
| Maisstärke | 25 | 15 |
| Talk | 15 | 10 |
| Magnesiumstearat | 2,5 | - |
| | 400 | 400 |

**Patentansprüche** für die Vertragsstaaten BE, CH, DE, FR, GB, IT, LI, NL, SE

1. 2-[2-(2-Hydroxy-3-tert.-butylamino-propoxy)-5-acylamino-phenyl]-1,3,4-oxadiazole der allgemeinen Formel I

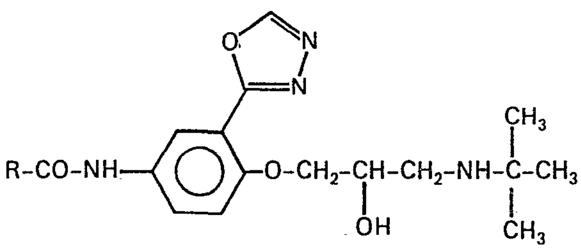

(I)

in der R eine geradkettige oder verzweigtkettige Alkylgruppe mit 1 bis 17 Kohlenstoffatomen oder eine Cycloalkylgruppe mit 3 bis 6 Kohlenstoffatomen darstellt, sowie deren physiologisch verträgliche Säureadditionssalze.

2. Verbindungen der allgemeinen Formel I gemäss Anspruch 1, in der R eine geradkettige oder verzweigtkettige Alkylgruppe mit 5 bis 10 Kohlenstoffatomen darstellt.

3. Verbindungen der allgemeinen Formel I gemäss Anspruch 1, in der R die n-Pentylgruppe, n-Hexylgruppe, n-Heptylgruppe, n-Octylgruppe, n-Nonylgruppe oder n-Decylgruppe darstellt.

4. 2-[2-(2-Hydroxy-3-tert.-butylamino-propoxy)-5-n-heptanoylaminophenyl]-1,3,4-oxadiazol.

5. 2-[2-(2-Hydroxy-3-tert.-butylamino-propoxy)-5-n-octanoylaminophenyl]-1,3,4-oxadiazol.

6. 2-[2-(2-Hydroxy-3-tert.-butylamino-propoxy)-5-acetamidophenyl]-1,3,4-oxadiazol.

7. Verfahren zur Herstellung der Verbindungen nach Anspruch 1, dadurch gekennzeichnet, dass man substituierte Oxadiazole der allgemeinen Formel II

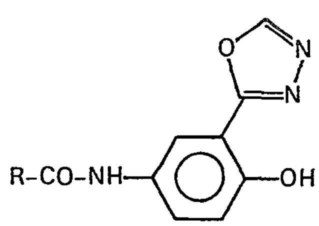

(II)

in der R die oben angegebene Bedeutung hat, mit Epichlorhydrin oder Epibromhydrin zu einem Gemisch von Verbindungen der allgemeinen Formeln III und IV umsetzt

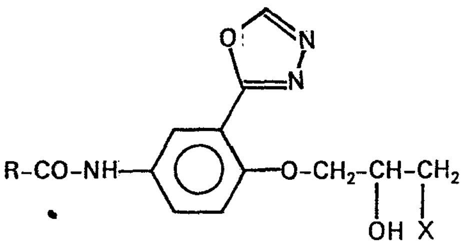

(III)

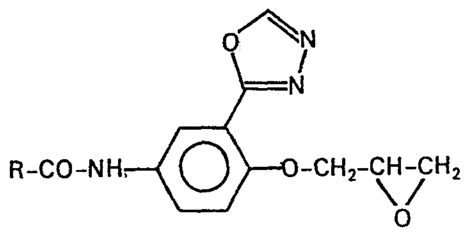

(IV)

worin R die oben angegebene Bedeutung hat und X für ein Chlor- oder Bromatom steht, und dieses anschliessend mit tert.-Butylamin zu Verbindungen der allgemeinen Formel I umsetzt sowie gegebenenfalls die freien Basen mit Säuren in die entsprechenden Säureadditionssalze umwandelt.

8. Pharmazeutisches Präparat, dadurch gekennzeichnet, dass es eine oder mehrere der Verbindungen gemäss Anspruch 1 und gegebenenfalls übliche Trägerstoffe und/oder Verdünnungsmittel enthält.

9. Verbindungen der allgemeinen Formel I oder deren physiologisch verträglichen Säureadditionssalze gemäss Anspruch 1 zur Anwendung bei Angina pectoris, Hypertonie und Arrhythmien.

10. 2-[2-(2-Hydroxy-3-tert.-butylamino-propoxy)-5-n-heptanoyl-aminophenyl]-1,3,4-oxadiazol oder dessen physiologisch verträglichen Säureadditionssalze zur Anwendung bei Angina pectoris, Hypertonie und Arrhythmien.

**Patentansprüche** für den Vertragsstaat AT

1. Verfahren zur Herstellung von neuen 2-[2-(2-Hydroxy-3-tert.-butylamino-propoxy)-5-acylamino-phenyl]-1,3,4-oxadiazolen der allgemeinen Formel I

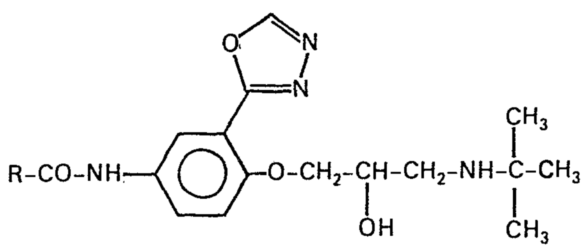

(I)

in der R eine geradkettige oder verzweigtkettige Alkylgruppe mit 1 bis 17 Kohlenstoffatomen oder eine Cycloalkylgruppe mit 3 bis 6 Kohlenstoffatomen darstellt, sowie deren physiologisch verträgliche Säureadditionssalzen, dadurch gekennzeichnet, dass man substituierte Oxadiazole der allgemeinen Formel II

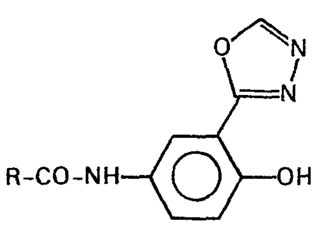

(II)

in der R die oben angegebene Bedeutung hat, mit Epichlorhydrin oder Epibromhydrin zu einem Gemisch von Verbindungen der allgemeinen Formeln III und IV umsetzt

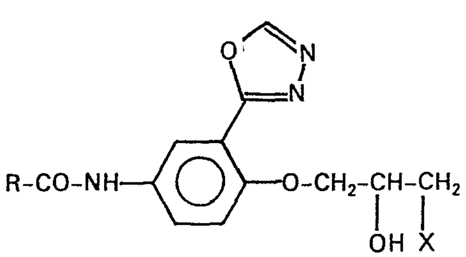

(III)

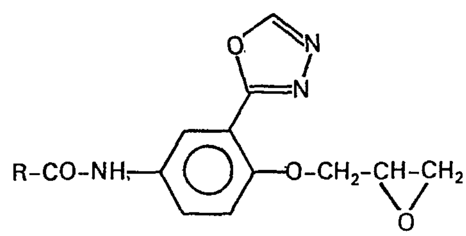

(IV)

worin R die oben angegebene Bedeutung hat und X für ein Chlor- oder Bromatom steht, und dieses anschliessend mit tert.-Butylamin zu Verbindungen der allgemeinen Formel I umsetzt sowie gegebenenfalls die freien Basen mit Säuren in die entsprechenden Säureadditionssalze umwandelt.

2. Verfahren zur Herstellung von 2-[2-(2-Hydroxy-3-tert.-butylamino-propoxy)-5-n-heptanoylaminophenyl]-1,3,4-oxadiazol nach Anspruch 1, dadurch gekennzeichnet, dass man 2-(2-Hydroxy-5-n-heptanoylaminophenyl]-1,3,4-oxadiazol mit Epichlorhydrin oder Epibromhydrin umsetzt und das Reaktionsprodukt mit tert.-Butylamin umsetzt.

**Claims** for the contracting states: BE, CH, DE, FR, GB, IT, LI, NL, SE

1. 2-[2-(2-Hydroxy-3-tert.-butylamino-prop-

oxy)-5-acylaminophenyl]-1,3,4-oxadiazoles of the general formula I

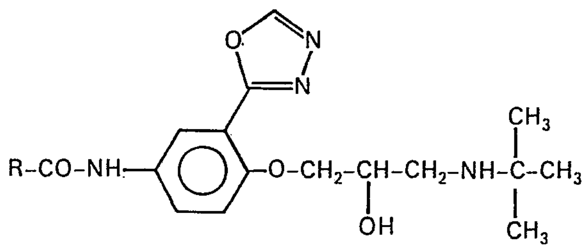

(I)

in which R represents a straight-chain or branched alkyl group with 1 to 17 carbon atoms or a cycloalkyl group with 3 to 6 carbon atoms, and physiologically acceptable acid addition salts thereof.

2. Compounds of the general formula I according to Claim 1, in which R represents a straight-chain or branched alkyl group with 5 to 10 carbon atoms.

3. Compounds of the general formula I according to Claim 1, in which R represents the n-pentyl group, n-hexyl group, n-heptyl group, n-octyl group, n-nonyl group or n-decyl group.

4. 2-[2-(2-Hydroxy-3-tert.-butylamino-propoxy)-5-n-heptanoylaminophenyl]-1,3,4-oxadiazole.

5. 2-[2-(2-Hydroxy-3-tert.-butylamino-propoxy)-5-n-octanoylaminophenyl]-1,3,4-oxadiazole.

6. 2-[2-(2-Hydroxy-3-tert.-butylamino-propoxy)-5-acetamidophenyl]-1,3,4-oxadiazole.

7. Process for the preparation of the compounds according to Claim 1, characterised in that substituted oxadiazoles of the general formula II

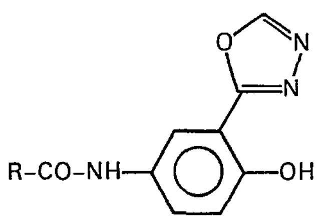

(II)

in which R has the abovementioned meaning, are reacted with epichlorohydrin or epibromohydrin to give a mixture of compounds of the general formulae III and IV

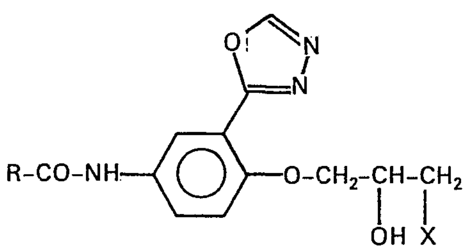

(III)

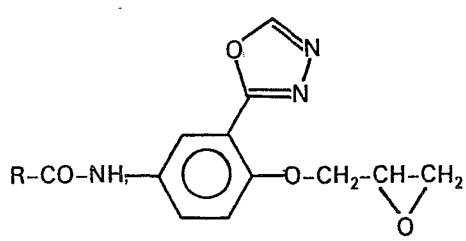

(IV)

wherein R has the abovementioned meaning and X represents a chlorine or bromine atom, and these are then reacted with tert.-butylamine to give compounds of the general formula I, and, if desired, the free bases are converted into the corresponding acid addition salts using acids.

8. Pharmaceutical product, characterised in that it contains one or more of the compounds according to Claim 1 and, where relevant, customary excipients and/or diluents.

9. Compounds of the general formula I or physiologically acceptable acid addition salts thereof, according to Claim 1, for use in cases of angina pectoris, hypertension and arrhythmias.

10. 2-[2-(2-Hydroxy-3-tert.-butylamino-propoxy)-5-n-heptanoyl-aminophenyl]-1,3,4-oxadiazole or physiologically acceptable acid addition salts thereof, for use in cases of angina pectoris, hypertension and arrhythmias.

**Revendications** pour les Etats contractants: BE, CH, DE, FR, GB, IT, LI, NL, SE

1. 2-[2-(2-Hydroxy-3-tert.-butylamino-propoxy)-5-acylaminophényl]-1,3,4-oxadiazoles de la formule générale I

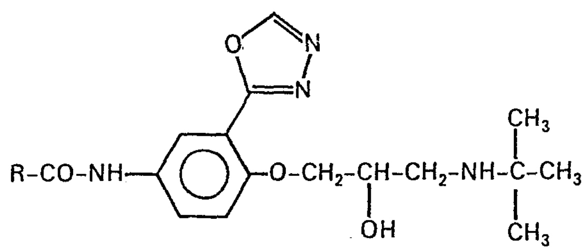

(I)

où R représente un radical alcoyle en chaîne droite ou ramifiée de 1 à 17 atomes de carbone ou un radical cycloalcoyle de 3 à 6 atomes de carbone, de même que leurs sels d'addition d'acides physiologiquement acceptables.

2. Composés de la formule générale I suivant la revendication 1, dans laquelle R représente un radical alcoyle en chaîne droite ou ramifiée de 5 à 10 atomes de carbone.

3. Composés de la formule générale I suivant la revendication 1, dans laquelle R représente le radical n-pentyle, n-hexyle, n-heptyle, n-octyle, n-nonyle ou n-décyle.

4. Le 2-[2-(2-hydroxy-3-tert.-butylamino-propoxy)-5-n-heptanoylaminophényl]-1,3,4-oxadiazole.

5. Le 2-[2-(2-hydroxy-3-tert.-butylamino-propoxy)-5-n-octanoylaminophényl]-1,3,4-oxadiazole.

6. Le 2-[2-(2-hydroxy-3-tert.-butylamino-propoxy)-5-acétamidophényl]-1,3,4-oxadiazole.

7. Procédé de préparation des composés suivant la revendication 1, caractérisé en ce qu'on fait réagir des oxadiazoles substitués de la formule générale II

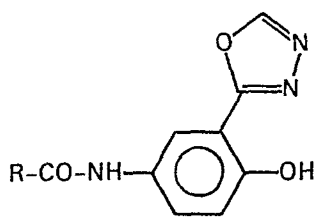

(II)

où R a la signification indiquée ci-dessus, avec l'épichlorhydrine ou l'épibromhydrine en un mélange de composés des formules générales III et IV

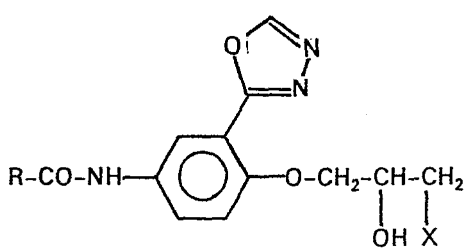

(III)

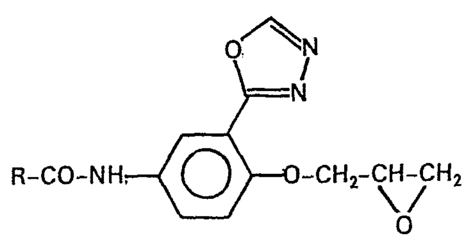

(IV)

où R a la signification indiquée ci-dessus et X représente un atome de chlore ou de brome, et on fait réagir ensuite celui-ci avec la t-butylamine en composés de formule générale I et on convertit éventuellement les bases libres au moyen d'acides en les sels d'addition d'acides correspondants.

8. Composition pharmaceutique, caractérisée en ce qu'elle contient un ou plusieurs des composés suivant la revendication 1 et éventuellement des excipients et/ou diluants habituels.

9. Composés de la formule générale I ou leurs sels d'addition d'acides physiologiquement acceptables suivant la revendication 1 pour l'administration en cas d'angine de poitrine, d'hypertension et d'arythmies.

10. Le 2-[2-(2-Hydroxy-3-tert.-butylamino-propoxy)-5-n-heptanoyl-aminophényl]-1,3,4-oxadiazole ou ses sels d'addition d'acides physiologiquement acceptables pour l'administration en cas d'angine de poitrine, d'hypertension et d'arythmies.

**Claims** for the Contracting state: AT

1. Process for the preparation of new 2-[2-(2-hydroxy-3-tert.-butylamino-propoxy)-5-acylamino-phenyl]-1,3,4-oxadiazoles of the general formula I

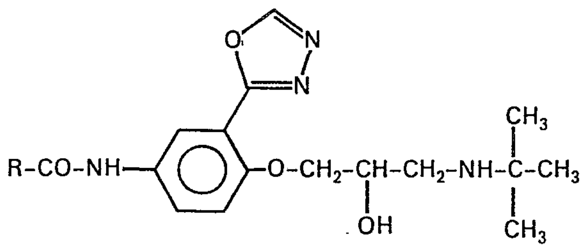

(I)

in which R represents a straight-chain or branched alkyl group with 1 to 17 carbon atoms or a cycloalkyl group with 3 to 6 carbon atoms, and physiologically acceptable acid addition salts thereof, characterised in that substituted oxadiazoles of the general formula II

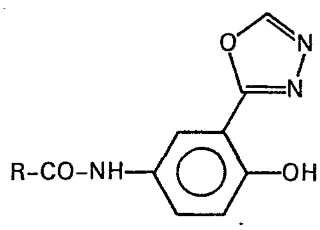

(II)

in which R has the abovementioned meaning, are reacted with epichlorohydrin or epibromohydrin to give a mixture of compounds of the general formulae III and IV

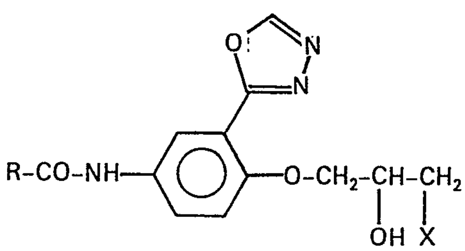

(III)

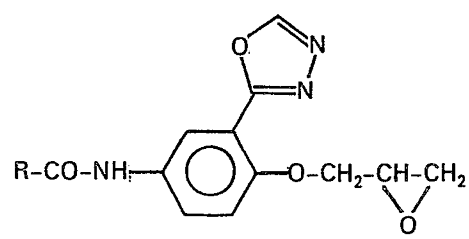

(IV)

wherein R has the abovementioned meaning and X represents a chlorine or bromine atom, and these are then reacted with tert.-butylamine to give compounds of the general formula I, and, if desired, the free bases are converted into the corresponding acid addition salts using acids.

2. Process for the preparation of 2-[2-(2-hydroxy-3-tert.-butylamino-propoxy)-5-n-heptanoylaminophenyl]-1,3,4-oxadiazole according to Claim 1, characterised in that 2-(2-hydroxy-5-n-heptanoylamino-phenyl)-1,3,4-oxadiazole is reacted with epichlorohydrin or epibromohydrin and the reaction product is reacted with tert.-butylamine.

**Revendications** pour l'Etat contractant: AT

1. Procédé de préparation de nouveaux 2-[2-(2-hydroxy-3-tert.-butylamino-propoxy)-5-acylamino-phényl]-1,3,4-oxadiazoles de la formule générale I

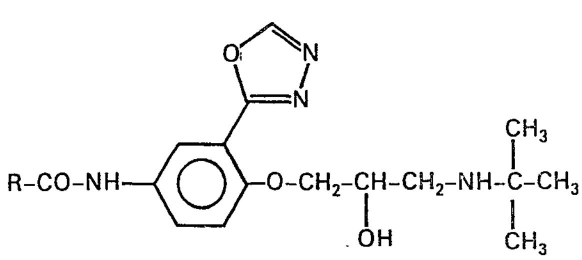

(I)

où R représente un radical alcoyle en chaîne droite ou ramifiée de 1 à 17 atomes de carbone ou un radical cycloalcoyle de 3 à 6 atomes de carbone, ainsi que de leurs sels d'addition d'acides physiologiquement acceptables, caractérisé en ce qu'on fait réagir des oxadiazoles substitués de formule générale II

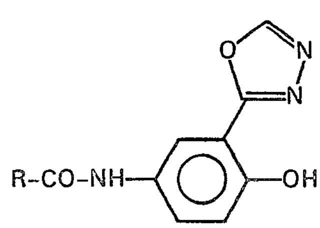

(II)

où R a la signification indiquée ci-dessus, avec l'épichlorhydrine ou l'épibromhydrine en un mélange de composés des formules III et IV

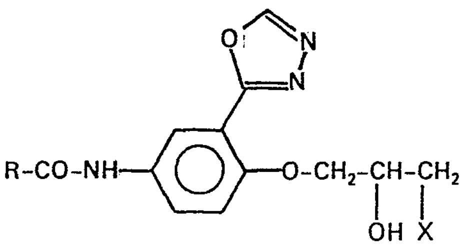

(III)

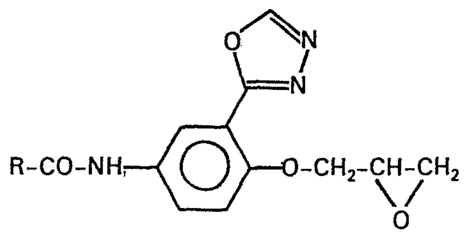

(IV)

où R a la signification indiquée ci-dessus et X représente un atome de chlore ou de brome, et on fait réagir ensuite celui-ci avec la t-butylamine en composés de formule générale I et on convertit éventuellement les bases libres au moyen d'acides en les sels d'addition d'acides correspondants.

2. Procédé de préparation du 2-[2-(2-hydroxy-3-tert.-butylamino-propoxy)-5-n-heptanoylaminophényl]-1,3,4-oxadiazole suivant la revendication 1, caractérisé en ce qu'on fait réagir le 2-(2-hydroxy-5-n-heptanoylamino-phényl]-1,3,4-oxadiazole avec l'épichlorhydrine ou l'épibromhydrine et on fait réagir le produit de réaction avec la t-butylamine.